# EUROPEAN PATENT SPECIFICATION

(11) **EP 1 493 401 B1**
(45) Date of publication and mention of the grant of the patent: **23.08.2006**
(21) Application number: 03077096.0
(22) Date of filing: 03.07.2003
(51) Int. Cl.: A61F 2/06

(54) **Hybrid stent apparatus**
Hybrid-Stentvorrichtung
Dispositif d'extenseur intravasculaire hybride

(43) Date of publication of application: 05.01.2005
(73) Proprietor: WILLIAM COOK EUROPE ApS, 4632 Bjaeverskov (DK); Cook Incorporated, Bloomington, Indiana 47404 (US)
(72) Inventor: Bassoe, Thomas, 4632 Bjaeverskov (DK)
(74) Representative: Garratt, Peter Douglas

(56) References cited:
- EP-A- 1 138 280
- US-A1- 2002 007 102
- US-A1- 2003 105 516
- US-B1- 6 241 746

## Description

### Technical field

The present invention relates to medical devices and in particular, to hybrid stent apparatus that is used in passageways of the human or animal body.

### Background of the invention

Balloon-expandable stents and self-expanding stents are well known in the art. A variety of materials is used to make stents, for example polymers, metals or shape memory alloys. Self-expanding stents are commonly made from stainless steel or shape-memory alloys, such as a nickel-titanium alloy, also known as Nitinol. Stents can be placed temporarily or permanently within a passageway of a human or animal body such as a duct, canal or blood vessel to aid healing or relieve an obstruction or stenosis.

In the case of balloon-expandable stents, the delivery system therefor has usually a relatively large diameter, particularly in the portion of the device where the balloon(s) is (are) located, because the balloon(s) has (have) to be folded when collapsed. Consequently, these devices tend to be relatively bulky and rigid, with limited lateral flexibility. Rigidity is nevertheless desirable if accurate positioning of a stent is to be achieved. It can be said that what a device for delivery of balloon-expandable stent(s) lacks in lateral flexibility is compensated by a higher level of placement accuracy. Balloon expandable stents have the drawback, particularly in coronary applications, of having to be initially overinflated and this causes trauma to the stented vessel. This drawback is further heightened at the ends of the stent where there is an abrupt change in radial force from the stented to the unstented tissue. This vascular trauma, called edge effect, can cause intimal hyperplasia and stenosis or closure of the vessel beyond the ends of the stent. The edge effect is particularly pronounced when a balloon expandable radioactive stent is deployed in an arterial stenosis, and is well known in coronary applications.

By contrast, devices for delivery of self-expanding stents are relatively less bulky than balloon expandable stents and therefore more laterally flexible, because there is no need to provide a delivery system that has means to expand the stent. Increased lateral flexibility of a device leads to an increased ability to negotiate tortuous vessels, but also to a relative lack of rigidity, which makes accurate positioning of the device in a blood vessel more difficult; as the health practitioner normally pushes on the proximal aspect of the device that is located outside the patient, increased lateral flexion of the distal part of the device that is located within a tortuous vessel of a patient may result instead of accurate forward movement. However, self-expanding stents and its less bulky architecture lends itself to smaller diameter vessel placement.

Hybrid stents were developed recently in an effort to provide a stent that combines the advantages of flexibility and accuracy of placement. US patent 6,315,708 to Salmon et al. discloses a hybrid stent that has a central section that is balloon-expandable and end sections that are self-expanding. The entire stent is mounted on a balloon of a balloon angioplasty catheter. A cylindrical elastomeric tube fitted around each of the self-expanding end sections of the stent retains the self-expanding sections of the stent in a collapsed state onto the balloon. Alternatively, the end sections of the Salmon stent are retained onto the stent delivery system by an outer sheath that is withdrawn by the health practioner in order to cause expansion of the end sections. The Salmon stent, because of the need to use means to expand the central section of the hybrid stent is substantially as bulky as a conventional balloon expandable stent, and has therefore rigidity similar to that of conventional balloon expandable stents. In summary, although the Salmon stent has several advantages over conventional stents, particularly with respect to reducing the occurrence of edge effects when used in the treatment of stenoses, its use is mainly indicated in cases where rigidity of the stent is preferred over flexibility, such as in the treatment of vascular stenoses. Salmon's stent is not suited for cases where flexibility of a stent is preferred over rigidity, as for example in the treatment of small and tortuous cerebral blood vessels. There is a need, in such cases, for stents and/or devices that are less bulky, of small diameter and where flexibility is preferred over rigidity.

The invention seeks to provide a hybrid stent apparatus mounted on a stent delivery device that can be used to catheterise tortuous arteries of a small diameter, that is highly flexible and yet that allows accurate placement of the stent apparatus to be deployed.

### Disclosure of the invention

According to one aspect of the present invention, there is provided a medical device as specified in claim 1. It solves the problem of providing a device that has excellent flexibility and that can be accurately placed, particularly in tortuous passageways of small diameter such as cerebral arteries. Accordingly, there is provided a hybrid stent apparatus with a self-expanding central part and balloon-expandable end parts. Because the central part of the hybrid stent apparatus is self-expanding, there is no need for a delivery system to provide means for deploying that part of the hybrid stent apparatus. This has the advantage of allowing the delivery system to be thin in the area corresponding to the central part of the hybrid stent apparatus, hence providing excellent flexibility to the present device. Control over the placement of the present device is also excellent, because expansion of the end parts of the hybrid stent apparatus is totally under the control of the health practioner who decides the exact locations at which to complete the deployment of the end parts. The deployed end parts of the hybrid stent provide a better grip on the wall vessel than the self-expanding central part of the stent. Furthermore, because the flexible hybrid stent is mounted on a delivery catheter, the latter adds desired rigidity to the present device allowing the health practioner to achieve deployment of the hybrid stent at a precise location within a passageway of a patient, without compromise to the desired flexibility of the present device. The present device has a further advantage in that it need not only be used in the treatment of stenoses, but also in the treatment of aneurysms. The maximum outer diameter of the expanded central part of a hybrid stent apparatus is naturally predetermined, and a hybrid stent can therefore be selected according to the diameter and length of an aneurysm to be treated, to provide an adequate lumen in the area of the dilated artery. Undesirable pressure on the dilated wall of the artery is therefore being kept to a minimum, and this is the reason why a hybrid stent apparatus with a balloon-expandable central part would not be suitable for the treatment of an aneurysm, as balloon dilatation of the central section of an hybrid stent increases the risk of causing undesirable barotrauma to an already weakened vessel wall. Furthermore, the length of the selected hybrid stent apparatus should be such as to allow the balloon-expandable end parts of the stent to be expanded within the healthy areas of the artery adjacent to each end of the aneurysm.

According to another aspect of the present invention, control over the expansion of the self-expanding part of the hybrid stent apparatus is achieved by the use of a constricting means, for example an outer sheath. Materials that can be used for the outer sheath are well known in the art and include polymers such as polyethylene, PET, polyurethane, polyamides, and polytetrafluoroethylene (PTFE). The wall of the outer sheath can be formed sufficiently thin to not substantially interfere with the flexibility of the present device, and not substantially increase its outer diameter.

According to yet another aspect of the present invention there is also provided a delivery catheter having a main lumen within which a guidewire can be moved, and an expandable arrangement, in this example at least one expandable balloon located at the distal portion of the catheter for expanding the end parts of the hybrid stent apparatus.

According to still another aspect of the present invention, there is provided a method of deploying a hybrid stent apparatus, through an appropriate access site, within a tubular passageway of a patient, such as an artery. Using for example the well-known Seldinger technique, whereby a balloon catheter having the present hybrid stent apparatus mounted at the distal portion thereof is introduced over a guidewire placed beforehand into a passageway of a patient, the hybrid stent apparatus is positioned at a desired location within an artery of a patient. Upon withdrawal of the outer sheath, the self-expanding central part of the hybrid stent apparatus expands radially, while the balloon-expandable end parts of the hybrid stent apparatus are only expanded when the balloon(s) of the balloon catheter is (are) expanded. At this stage of the procedure, the portions of the self-expanding central part of the hybrid stent situated immediately adjacent to each end part of the stent are prevented from fully expanding by the balloon-expandable end parts of the stent that are not yet deployed. At the same time, the portions of the end parts of the hybrid stent that are immediately adjacent to the self-expanding central part of the stent are partly expanded by the expansion of the adjacent central part of the hybrid stent. The end parts of the hybrid stent apparatus can therefore be expanded at a precise desired location, for example within the healthy parts of an artery adjacent to each end of a lesion such as an aneurysm, giving the device a high positioning accuracy. Because the balloon catheter need not have means to expand the self-expanding central part of the hybrid stent apparatus, the device is relatively thin and therefore more flexible than an equivalent device with a hybrid stent apparatus that has a balloon expandable central part. After deployment of the hybrid stent apparatus and removal of the components of the present device still left in the patient at this stage, such as the outer sheath and/or the guidewire, the access site is closed in a routine manner.

According to a final aspect of the present invention, there is also provided a method of deploying several hybrid stent apparatus to stent lesions having a length that is longer than the maximal available length of a single hybrid stent apparatus. Using the method disclosed in the previous paragraph, the distal end part of a first hybrid stent apparatus is expanded. The proximal end part of the first hybrid stent apparatus that is located within the diseased portion of the patient's passageway is then expanded to its nominal expanded diameter, either separately or simultaneously with the distal end part of the hybrid stent apparatus according to the type of balloon catheter used. The balloon catheter and outer sheath of the first hybrid stent apparatus are then completely withdrawn from the patient, but the guidewire is left in place. A second device comprising a hybrid stent apparatus of a desired length and diameter mounted on a balloon catheter is then introduced within the patient's passageway over the guidewire that was left in place. The position of the second device is selected to enable the health practitioner to expand, after withdrawal of the outer sheath of the second device, the distal end part of the second hybrid stent apparatus within the proximal end part of the firstly deployed hybrid stent apparatus. The proximal end part of the second hybrid stent apparatus is expanded either separately or simultaneously with the distal end part of the second hybrid stent apparatus, according to the type of balloon catheter used. If necessary, additional hybrid stent apparatus can be deployed within a diseased portion of a passageway by repeating the method disclosed above. Full deployment of a series of stents is achieved after the last stent of a linear series of stents is deployed within the desired locations of the patient's passageway. The outer sheath and balloon catheter of the last implanted device, and the guidewire are finally withdrawn from the patient and the access site is closed in a routine manner.

### Brief description of the drawings

The invention will now be described by way of example of carrying out the subject matter and with reference to the accompanying drawings in which:
FIG 1 is a longitudinal cross-section of a distal part of a device illustrating a hybrid stent apparatus mounted on a non-expanded single lumen balloon catheter and retained in a collapsed state by an outer sheath in accordance to an embodiment of the present invention; and
FIGs 2 to 4 are diagrammatic representations of different aspects of the parts of the hybrid stent apparatus in accordance to an embodiment of the present invention; and
FIG 5 is a longitudinal cross-section of the expanded single lumen balloon of catheter in accordance with an FIG 1 in accordance with a preferred aspect of the present invention; and
FIG 6 is a longitudinal cross-section of another expanded single lumen balloon catheter in accordance with another aspect of the present invention; and
FIG 7 is a longitudinal cross-section of an expanded dual lumen balloon catheter in accordance with another aspect of the present invention; and
FIG 8 is a longitudinal cross-section of an expanded single lumen balloon catheter in accordance with an aspect of the present invention; and
FIG 9 is a longitudinal cross-section of a distal part of the device shown in FIG 1 wherein the self-expanding middle part of the hybrid stent apparatus is expanded after removal of the outer sheath and before expansion of the balloon; and
FIG 10 is a longitudinal cross-section of a distal part of the device shown in FIGs 1 and 9 wherein the end parts of the hybrid stent apparatus are expanded by expansion of the balloon of FIG 5.

### Detailed description

The terms "hybrid stent apparatus" refer to a single stent apparatus having at least one part of a material different from the material of at least one other part of the stent apparatus. A hybrid stent apparatus can also be of a single material stent, i.e. all parts of the stent apparatus are of the same material, comprising at least one part that has at least one physical property, for example expansibility, different from that of at least one other part of the stent apparatus. Finally, the terms refer also to a multi-material stent apparatus as firstly defined above, further having at least one part differing from at least one other part of the hybrid stent apparatus by its physical property, for example expansibility. When considering the sides of a device or part thereof, the relative terms "proximal" and "distal" refer to the side of the device or part thereof located towards and away from the health practitioner manipulating the said device respectively.

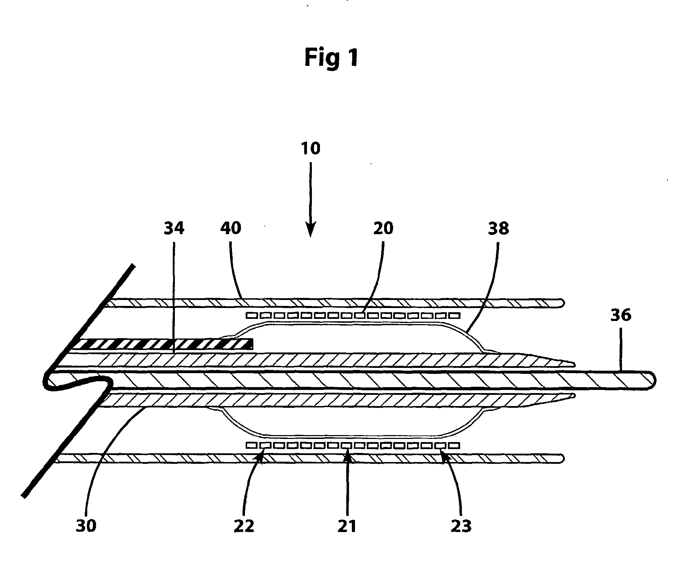

FIG 1 illustrates a first embodiment of device 10 comprising three components: a hybrid stent apparatus 20, mounted on means for delivering and expanding said hybrid stent apparatus, in this example a balloon catheter 30, and constricting means for holding said hybrid stent apparatus in a radially collapsed state, in this example an outer sheath 40.

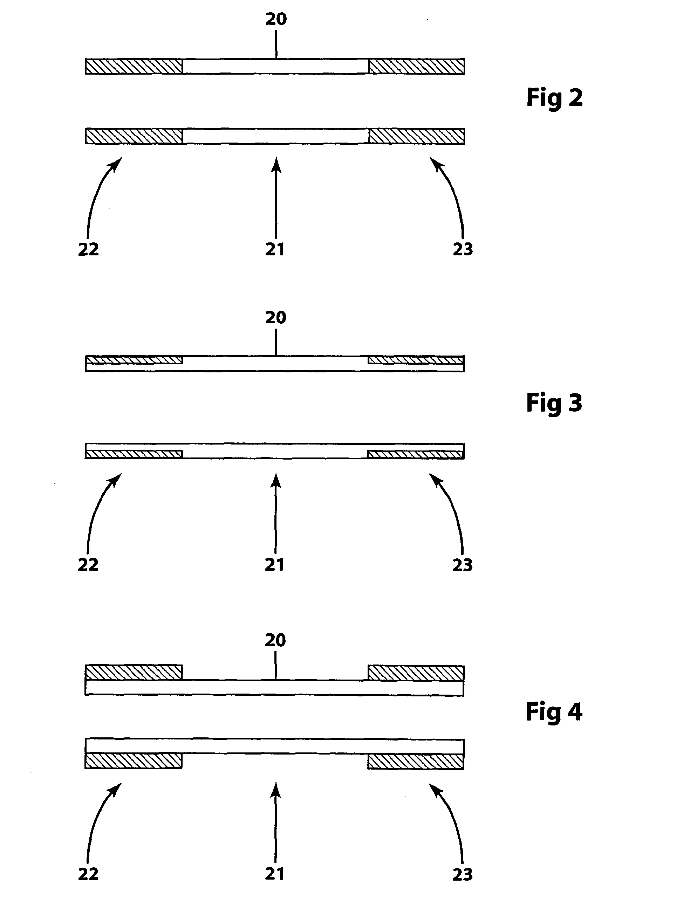

Hybrid stent apparatus 20 is preferably a coiled wire, but can be formed with any other pattern of one or several wires, the stent having a generally tubular shape in order to be introduced into a tubular passageway of a patient, for example an artery. In the present embodiment, the hybrid stent apparatus 20 comprises three parts as illustrated in FIGs 1 to 4: a central part 21 that is self-expanding, and end parts 22 and 23 that are balloon-expandable. Materials used for hybrid stent apparatus 20 include one or several of polymers, metals such as stainless steel, nickel, titanium, tantalum, and biocompatible metals and alloys thereof and in particular shape memory alloys such as nickel-titanium alloys, also known as Nitinol. The shape memory property of Nitinol can advantageously be used to provide different expansibility properties, such as self-expansion, to parts of the present hybrid stent apparatus. Details of the method of imparting shape memory property to a Nitinol wire are well known in the art and will not be described in the present application. Generally, the method consists in setting the transition temperature of a hybrid stent apparatus or part thereof to be treated, just below or well above body temperature. The transition temperature, or in particular its austenitic final (A_{f}) temperature, is the temperature above which all of the heat-treated and deformed Nitinol wire returns to its preset or original shape (austenitic state). When the transition temperature is set just below body temperature, the Nitinol wire of the hybrid stent apparatus will revert to its preset shape only after it has been introduced in a patient, i.e. the hybrid stent apparatus expands to its preset expanded diameter. When the transition temperature is set well above body temperature, the hybrid stent apparatus does not self-expand and does not change its current shape after it has been introduced into a patient, i.e. the hybrid stent apparatus retains its deformed shape (martensitic state). Accordingly, the present hybrid stent apparatus comprises a central part that has a transition temperature set just below body temperature and above normal room temperature. The stent has a radially collapsed state at room temperature and reverts spontaneously to a preset expanded state after introduction into a blood vessel of a patient. The end parts of the present hybrid stent apparatus have a transition temperature set well above body temperature, and therefore will not expand spontaneously to a larger diameter after introduction into the blood vessel of a patient. However, martensitic Nitinol has very little elasticity or springiness. Thus, the martensitic Nitinol can be very easily deformed and does pulse with blood flow. The end parts 22, 23 of the hybrid stent apparatus 20 need therefore to be expanded with a balloon, as disclosed below. Other materials can be used for the hybrid stent apparatus, for example the present hybrid stent apparatus can be formed by having a Nitinol central part and stainless steel end parts bonded, mechanically linked or soldered to the central part as illustrated in FIG 2. In the other examples of FIGs 3 and 4, a layer of Nitinol is present in all the parts of the hybrid stent apparatus, with the end parts having additionally a layer of another material, for example stainless steel, outside the Nitinol layer. In the example of FIG 3, the total thickness of the layers constituting the end parts of the hybrid stent apparatus is substantially equal to the thickness of the central part. In the example of FIG 4, the thickness of the Nitinol layer is substantially constant throughout the entire hybrid stent apparatus and the layer of the other material, for example stainless steel, is simply added outside the layer of Nitinol at the end parts. If desired, the thickness of the layer of the central part could be formed thicker than that of the end parts of the hybrid stent apparatus. Other materials and combinations of materials will be readily apparent to the skilled person keeping in mind that the end parts 22, 23 of the hybrid stent apparatus are balloon-expandable and the central part is self-expanding. Any one embodiment of the hybrid stent apparatus of the present application can also be a radioactive stent or a stent coated with or comprising in its structure, or in part(s) of its structure, at least one bioactive agent to be released in the patient after deployment of the hybrid stent apparatus.

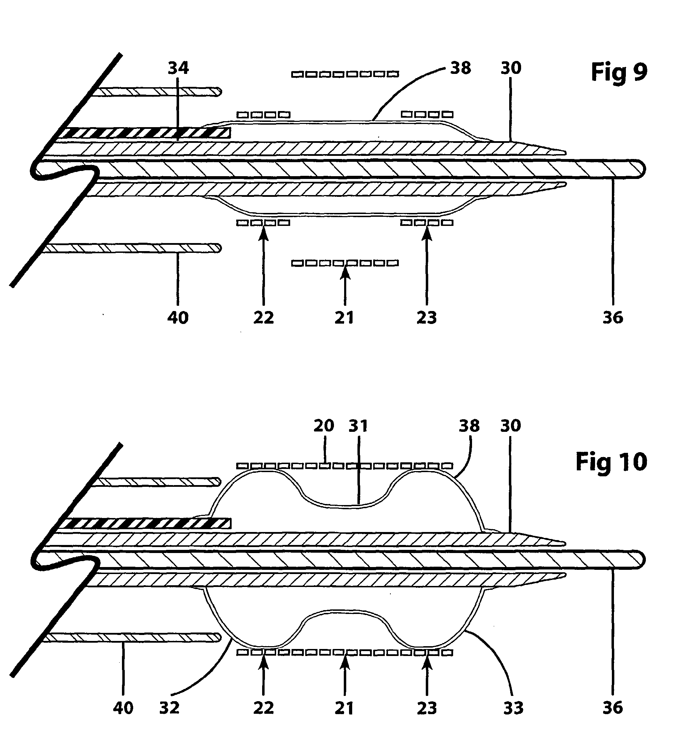

In FIG 1, outer sheath 40 maintains the hybrid stent apparatus 20, mounted at the distal part of catheter 30, in a radially collapsed state. The material of the outer sheath, in this example, is preferably a polymer or copolymer material such as polytetrafluoroethylene (PTFE), PET, polyethylene or polyurethane, well-known materials in the art. Any other biocompatible material that is highly resistant to tearing could be used. Any such type of material offers the advantage that the outer sheath can be very thin and retain its high resistance to tearing. Outer sheath 40 is slidable over the mounted hybrid stent apparatus 20 and balloon catheter 30, and withdrawal of the outer sheath 40 enables the self-expanding central part 21 of the hybrid stent apparatus to expand radially as illustrated in FIG 9. Optionally, a lubricant or hydrophilic material can be used on the inner surface of the outer sheath 40 to enable easier withdrawal of the outer sheath after device 10 has been positioned at a desired location in an artery of a patient.

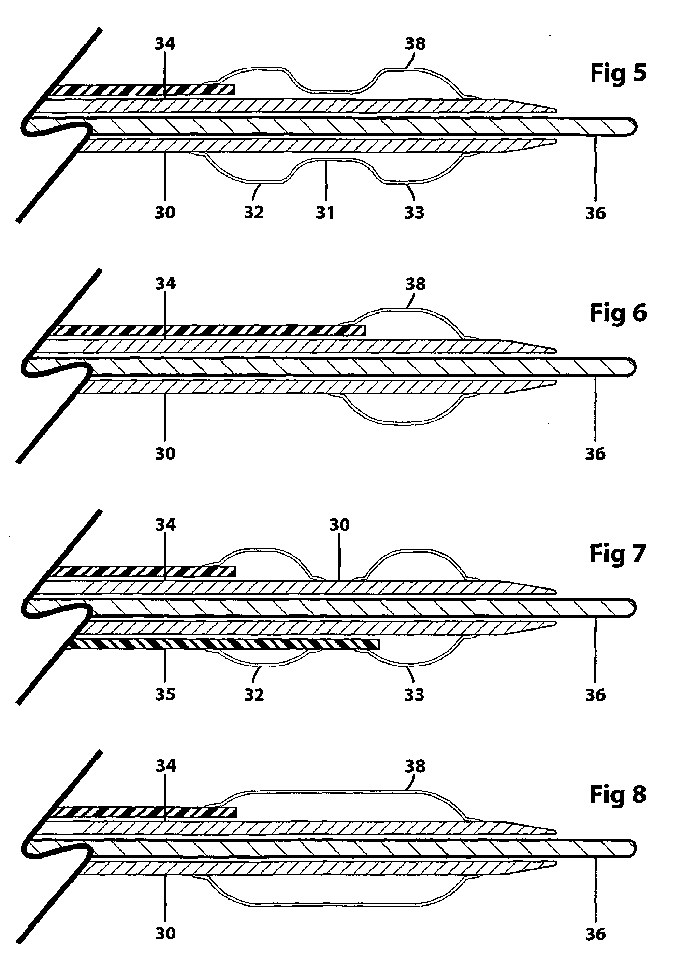

Catheter 30 of FIG 1 has a radially expandable arrangement located at its distal portion, in this example a single lumen balloon 38 for expanding the hybrid stent apparatus mounted at said distal portion. Inflation lumen 34 is connected to commercially available means for delivery of fluid under pressure in order to enable radial expansion of balloon 38. Although longitudinal inflation lumen 34 in each of FIG 1 and FIGs 5 to 10 is represented externally adjacent to catheter 30, inflation lumen 34 could be formed within the wall of catheter 30. The material of balloon 38, in this example is a polymer such as nylon 12 and is well known in the art for its high tensile strength and non-distendable property, making it an excellent material for dilatation balloons. Balloon 38 may have one of several shapes, as illustrated in FIGs 5 to 8, the balloons being formed at specific locations along the length of the distal portion of the catheter 30, to enable expansion of the end sections 22 and 23 of the hybrid stent apparatus 20 when mounted onto balloon-catheter 30. In the preferred aspect of the present invention, as illustrated in FIG 5, the single lumen balloon catheter 38 has, when radially expanded, a recessed central section 31 that substantially corresponds to the central part 21 of hybrid stent apparatus 20. The part of the device substantially corresponding to the central part of hybrid stent apparatus 20 and recessed central section of balloon 38 is therefore relatively thin and flexible, because the central section of balloon 38 does not need to be folded when the collapsed hybrid stent apparatus is mounted onto balloon catheter 30. When hybrid stent apparatus 20 is mounted on balloon catheter 30 of FIG 5, the end parts 22, 23 of hybrid stent apparatus 20 match the end sections 32, 33 of balloon 38. Expanding balloon 38 causes end parts 22, 23 of hybrid stent apparatus 20 to expand radially and simultaneously, as illustrated in Figure 10. However, because the expanded diameter of central section 31 of balloon 38 is significantly less than the diameter of the expanded central part 21 of hybrid stent apparatus 20, balloon 38 and the central part of hybrid stent apparatus 20 do not normally contact at any time after self-expansion of the central part 21 of hybrid stent apparatus 20, as shown in FIG 10. In FIG 6, balloon 38 is also a single lumen balloon, but its length matches the length of one of the distal sections 22, 23. In the embodiment of FIG 6, the end parts 22, 23 are substantially of equal length. After withdrawal of outer sheath 40, which results in self-expansion of central part 21 of hybrid stent apparatus 20, end parts 22 and 23 have to be expanded sequentially by balloon 38. FIG 7 illustrates a dual balloon catheter 30, wherein each balloon 32, 33 has an independent lumen continuous with individual inflation lumina 34 and 35. Alternatively, the inflation lumen 34 can be common to both balloons 32 and 33, in which case expansion of balloon 32 and 33 causes substantially simultaneous expansion of end parts 22, 23 of hybrid stent apparatus 20. Preferably, in the aspect of FIG 7, each balloon lumen is continuous with an individual inflation lumen, enabling separate and precise control of the expansion of each balloon 32, 33. Although the longitudinal inflation lumina 34 and 35 of FIG 7 are represented externally adjacent to catheter 30, inflation lumina 34 and/or 35 could be formed within the wall of catheter 30. Balloon 38 of FIG 8 is a single lumen balloon that has substantially the same length as that of the hybrid stent apparatus 20. Although expansion of balloon 38, after withdrawal of outer sheath 40, causes the end parts 22, 23 of hybrid stent apparatus 20 to expand radially and simultaneously, the central part 21 of the stent is substantially not affected by the balloon dilatation because upon removal of the outer sheath 40, the central part 21 self-expands to its preset maximum diameter that is substantially equal to the maximum diameter of the expanded balloon 38. Device 10 corresponding to the embodiment of FIG 8 is, as a result of the balloon configuration, bulkier than any of the devices corresponding to the embodiments of FIGs 5 to 7 because collapsed balloon 38 has to be folded when the hybrid stent apparatus is mounted onto catheter 30 within the outer sheath 40, resulting in some loss of lateral flexibility of the device 20 when compared to the other embodiments of FIGs 5 to 7.

As illustrated in FIG 1, balloon catheter 30 has a lumen into which guidewire 36 can be moved. The health practitioner can use guidewire 36, previously introduced into a desired blood vessel of a patient, to guide balloon catheter 30, with mounted hybrid stent apparatus 20 thereon, over guidewire 36 into a desired passageway or artery. Guidewire 36 can be withdrawn from the main lumen of the balloon catheter 30 and the lumen can be used to deliver desired biocompatible agents into the blood circulation, or to remove a quantity of blood, for example for diagnostic purposes. Naturally, the guidewire can also be withdrawn at a later stage of the procedure.

The distal portion of the balloon catheter 30 can be formed to have any shape. For example, the distal end of catheter 30 can be opened or closed. In either case, the distal portion of the balloon catheter 30 can be shaped to taper progressively towards its distal end, or can be formed to have any desired shape, as would be readily apparent to the skilled person. A gradual tapering of the distal portion of the balloon catheter 30, distal to balloon 38, would present the advantage of progressive increased flexibility of the distal portion towards the distal end of catheter 30, increasing therefore the ability of the catheter to track the lumen of tortuous blood vessels. The tapering of the distal portion of the balloon catheter 30 is very gradual and without abrupt transitions in order to avoid affecting adversely the trackability of the device. Alternatively, a soft tip formed from an appropriate material, for example silicone, can be bonded to the distal end of the balloon catheter 30 by any method well known in the art, such as heat or chemical bonding. The soft tip can also have any desired shape, such as tapered for example. Furthermore, device 20 of any of the above embodiments may have at least one side hole formed into the wall of catheter 30, preferably in its distal portion to allow communication between the main lumen of the catheter and that of the blood vessel at one or several desired locations. At least one extra lumen, separate from the main lumen of the balloon catheter 30 and inflation lumen 34 or 35, can be formed in the balloon catheter 30 for the delivery of drugs, contrast agents, saline, etc. into the blood circulation via at least one separate channel.

Indicia, detectable by appropriate imaging methods such as conventional radiography, fluoroscopy or ultrasound, can be incorporated at any specific location(s) along catheter 30 and/or balloon 38, in order to enable the health practitioner to position the balloon catheter 30 at a precise desired location within a blood vessel.

In order to clarify the relationship between the structure of device 10 and its function, a summary of a method used to implant the hybrid stent apparatus in an artery of a patient is presented below. Device 10 is supplied in a sterile package and comprises a hybrid stent apparatus 20 having one of a range of available lengths between about 10 mm to about 40 mm, and one of a range of available outer diameters of the expanded hybrid stents between 2 mm and 5 mm in 0.5 mm increments. The outer diameter of the device 10 of FIG 1, comprising the collapsed hybrid stent 20, is about 0.5 mm. An access site to a desired artery of a patient and the patient himself are prepared according to procedures in effect at individual medical centres. Catheterisation is done by the well-established Seldinger technique of firstly introducing a guidewire into a blood vessel of the access site of the patient, followed by positioning of the guidewire in the desired artery. The catheter is then introduced into the patient via the access site and moved over the guidewire until the hybrid stent apparatus, mounted on the distal portion of the balloon catheter 30, is located at a desired position within an artery to be stented. Radiographic or other imaging methods can be used at any time to ascertain the position of the catheter and hybrid stent apparatus with respect to anatomical landmarks and/or an arterial lesion to be stented. The health practitioner can rely also, if desired, on contrast studies obtained by injecting a contrast agent in the artery of the patient where the device is located, through the main lumen or a separate channel of balloon catheter 30. Once the distal portion of device 10 is judged to be in its final position, the outer sheath 40 is slowly withdrawn while catheter 30 is maintained in its present position, allowing the distal end part 23 of hybrid stent apparatus 20 to be uncovered, but not to expand in the arterial lumen at this stage of the procedure. The central part 21 of hybrid stent apparatus 20 self-expands radially, from a distal to a proximal direction as the outer sheath 40 is progressively withdrawn from the patient. Further withdrawal of the outer sheath allows the proximal end part 22 of hybrid stent apparatus 20 to be uncovered, but not to expand in the arterial lumen as illustrated in FIG 9. Expansion of the end parts 22, 23 of hybrid stent apparatus 20 is accomplished by radially expanding balloon 38. When balloon catheter 30 comprises a single lumen balloon 38 of a length substantially equal to the length of the hybrid stent apparatus 20, or a dual lumen balloon 32, 33 with a single common inflation lumen, expansion of the end parts 22, 23 is substantially simultaneous as illustrated in FIG 10. In case the present method is used to treat an aneurysm, it has to be insured that after deployment of the hybrid stent apparatus, the end parts 22 and 23, contact the healthy parts of the arterial wall adjacent to respectively the proximal and distal limit of the aneurysm to be stented. When balloon catheter 30 is a single lumen balloon of a length substantially equal to the length of one of the end parts of hybrid stent apparatus 20, the expansion of the end parts of hybrid stent apparatus 20 is sequential, i.e. the first end part to be expanded can be either the proximal part 22 or the distal part 23. After expansion of the end parts, the balloon catheter 30, the outer sheath 40 and the guidewire 36 are withdrawn from the patient and the access site is closed in a routine manner.

## Claims

1. A medical device (10) for use in a passageway of a human or animal body, comprising a catheter (30) having a lumen extending longitudinally therethrough and through which a guidewire (36) can be passed, said catheter (30) having an expandable arrangement located at a distal portion of the catheter, said distal portion of the catheter being laterally flexible, a hybrid stent (20) mounted at said distal portion of the catheter, and an outer sheath (40) slidably positioned over the hybrid stent (20) for constricting the said stent in a collapsed condition, **characterised in that** the stent (20) has a middle part (21) that is self-expanding and end parts (22, 23) that are expandable by the expandable arrangement.

2. A medical device (10) as claimed in claim 1, wherein the expandable arrangement is a single lumen balloon (38) having a length substantially equal to the length of stent apparatus (20) and having a recessed central section corresponding to the central part of stent apparatus (20).

3. A medical device (10) as claimed in claim 1, wherein the expandable arrangement is a single lumen balloon (38) having a length substantially equal to the length of one of the end parts (22, 23) of stent apparatus (20).

4. A medical device (10) as claimed in claim 1, wherein the expandable arrangement is a single lumen balloon (38) having a length substantially equal to the length of stent apparatus (20).

5. A medical device (10) as claimed in claim 1, wherein the expandable arrangement comprises two balloons (32, 33), each balloon having a length substantially equal to the length of the corresponding end part (22, 23) of stent apparatus (20).

6. A medical device (10) as claimed in claim 5, wherein each balloon has a separate inflation lumen (34, 35).

7. A medical device (10) as claimed in claim 5, wherein the balloons have a common inflation lumen (34).

8. A medical device as claimed in any one of the preceding claims, wherein the material of each part of the stent apparatus (20) is a metal.

9. A medical device as claimed in any one of the preceding claims, wherein the material of each part of the stent apparatus (20) is stainless steel.

10. A medical device as claimed in any one of claims 1 to 8, wherein the material of each part of the stent apparatus (20) is a shape memory metal.

11. A medical device as claimed in any one of claims 1 to 8, wherein the material of the stent apparatus (20) is stainless steel and a shape memory metal.

12. A medical device as claimed in claim 10 or 11, wherein the shape memory metal is Nitinol.

13. A medical device as claimed in any one of the preceding claims, wherein the material of the balloon(s) (38) is polytetrafluoroethylene.

14. A medical device as claimed in any one of the preceding claims, wherein the material of the outer sheath (40) is polytetrafluoroethylene.

## Patentansprüche

1. Medizinische Vorrichtung (10) zur Verwendung in einem Durchgang (passageway) eines menschlichen Körpers oder eines Tierkörpers, mit einem Katheter (30), der ein Lumen aufweist, das sich in Längsricnhtung durch ihn hindurch erstreckt und durch das ein Führungsdraht (36) schiebbar ist, wobei der Katheter (30) an einem distalen Abschnitt eine aufweitbare Anordnung aufweist, wobei der distale Abschnitt des Katheters zur Seite hin flexibel ist, einem Hybrid-Stent (20), der an dem distalen Abschnitt des Katheters angebracht ist, und einer Außenhülle (40), die gleitend über dem Hybrid-Stent (20) positioniert ist, um den Stent in einem kollabierten Zustand einzugrenzen, **dadurch gekennzeichnet, dass** der Stent (20) einen Mittelteil (21) aufweist, der sich selbst aufweitet, sowie Endteile (22, 23), die durch die aufweitbare Anordnung aufgeweitet werden können.

2. Medizinische Vorrichtung (10) nach Anspruch 1, wobei die aufweitbare Anordnung ein einziger Lumen-Ballon (38) ist, dessen Länge im Wesentlichen gleich der Länge der Stentvorrichtung (20) ist, und der einen vertieften Mittelabschnitt aufweist, der dem Mittelteil der Stentvorrichtung (20) entspricht.

3. Medizinische Vorrichtung (10) nach Anspruch 1, wobei die aufweitbare Anordnung ein einziger Lumen-Ballon (38) ist, dessen Länge im Wesentlichen gleich der Länge eines der Endteile (22, 23) der Sientvorrichtung (20) ist.

4. Medizinische Vorrichtung (10) nach Anspruch 1, wobei die aufweitbare Anordnung ein einziger Lumen-Ballon (38) ist, dessen Länge im Wesentlichen gleich der Länge der Stentvorrichtung (20) ist.

5. Medizinische Vorrichtung (10) nach Anspruch 1, wobei die aufweitbare Anordnung zwei Ballone (32, 33) aufweist, deren Länge jeweils im Wesentlichen gleich der Länge des entsprechenden Endteils (22, 23) der Stentvorrichtung (20) ist.

6. Medizinische Vorrichtung (10) nach Anspruch 5, wobei jeder Ballon ein separates Aufblas-Lumen (34, 35) aufweist.

7. Medizinische Vorrichtung (10) nach Anspruch 5, wobei die Ballone ein gemeinsames Aufblas-Lumen (34) aufweisen.

8. Medizinische Vorrichtung nach einem der vorhergehenden Ansprüche, wobei das Material jedes Teils der Stentvorriohtung (20) ein Metall ist.

9. Medizinische Vorrichtung nach einem der vorhergehenden Ansprüche, wobei das Material jedes Teils der Stentvorrichtung (20) rostfreier Stahl ist.

10. Medizinische Vorrichtung nach einem der Ansprüche 1 bis 8, wobei das Material jedes Teils der Stentvorrichtung (20) ein Formgedächtnismeiall ist.

11. Medizinische Vorrichtung nach einem der Ansprüche 1 bis 8, wobei das Material der Stentvorrichtung (20) rostfreier Stahl und ein Formgedächtnismetall ist.

12. Medizinische Vorrichtung nach Anspruch 10 oder 11, wobei das Fonngedächtnismetall Nitinol ist.

13. Medizinische Vorrichtung nach einem der vorhergehenden Ansprüche, wobei das Material des Ballons /der Ballone (38) Polytetrafluorethylen ist.

14. Medizinische Vorrichtung nach einem der vorhergehenden Ansprüche, wobei das Material der Außenhülle (40) Polytetrafluorethylen ist.

## Revendications

1. Dispositif médical (10) destiné à être utilisé dans un passage d'un corps humain ou animal, comprenant un cathéter (30) ayant une lumière s'étendant longitudinalement à travers celui-ci et à travers laquelle un fil de guidage (36) peut être passé, ledit cathéter (30) ayant un arrangement extensible situé an niveau d'une partie distale du cathéter, ladite partie distale du cathéter étant flexible latéralement, un-extenseur intravasculaire hybride (20) monté au niveau de ladite partie distale du cathéter, et. une gaine externe (40) positionnée de manière à pouvoir glisser sur ledit extenseur intravasculaire hybride (20) pour restreindre ledit extenseur intravasculaire en condition écrase, **caractérise en ce que** l'extenseur intravasculaire (20) présente une partie médiane (21) auto-extensible et des parties terminales (22, 23) qui sont extensibles par l'arrangement extensible.

2. Dispositif médical (10) selon la revendication 1, dans lequel l'arrangement extensible est un ballonnet (38) à une seule lumière ayant une longueur sensiblement égale à la longueur du dispositif d'extenseur intravasculaire (20) et ayant une partie centrale en creux correspondant à la partie centrale du dispositif d'extenseur intravasculaire (20).

3. Dispositif médical (10) selon la revendication 1, dans lequel l'arrangement extensible et un ballonnet (38) à une seule lumière ayant une longueur sensiblement égale à la longueur de l'une des parties terminales (22, 23) du dispositif d'extenseur intravasculaire (20).

4. Dispositif médical (10) selon la revendication 1, dans lequel l'arrangement extensible est un ballonnet (38) à une seule lumière ayant une longueur sensiblement égale à la longueur du dispositif d'extenseur intravasculaire (20).

5. Dispositif médical (10) selon la revendication 1, dans lequel l'arrangement extensible comprend deux ballonnets (32, 33), chaque ballonnet ayant une longueur sensiblement égale à la longueur de la partie terminale correspondante (22, 23) du dispositif d'extenseur intravasculaire (20).

6. Dispositif médical (10) selon la revendication 5, dans lequel chaque ballonnet présente une lumière de gonflage distincte (34, 35).

7. Dispositif médical (10) selon la revendication 5, dans lequel les ballonnets présentent une lumière de gonflage commune (34).

8. Dispositif médical selon l'une quelconque des revendications précédentes, dans lequel le matériau de chaque partie du dispositif d'extenseur intravasculaire (20) est du métal.

9. Dispositif médical selon l'une quelconque des revendications précédentes, dans lequel le matériau de chaque partie de dispositif d'extenseur intravasculaire (20) est de l'acier inoxydable.

10. Dispositif médical selon l'une quelconque des revendications 1 à 8, dans lequel le matériau de chaque partie du dispositif d'extenseur intravasculaire (20) est du métal à mémoire de forme.

11. Dispositif médical selon l'une quelconque des revendications 1 à 8, dans lequel le matériau du dispositif d'extenseur intravasculaire (20) est de l'acier inoxydable et un métal à mémoire de forme.

12. Dispositif médical selon la revendication 10 ou la revendication 11, dans lequel le métal à mémoire de forme est du nitinol.

13. Dispositif médical selon l'une quelconque des revendications précédentes, dans lequel le matériau du ou des ballonnets (38) est du polytétrafluoroéthylène.

14. Dispositif médical selon l'une quelconque des revendications précédentes, dans lequel le matériau de la gaine externe (40) est du polytétrafluoroéthylène.
